# EUROPEAN PATENT APPLICATION

(11) **EP 2 515 108 A1**
(43) Date of publication of application: **24.10.2012**
(21) Application number: 12164878.6
(22) Date of filing: 20.04.2012
(51) Int. Cl.: G01N 33/00, G02B 27/01, G08B 17/117, G08B 21/16, G08B 21/04

(54) **Methods and Systems for Use in Monitoring Hazardous Gases**

(30) Priority: 21.04.2011 US 201113091794
(71) Applicant: General Electric Company, Schenectady, NY 12345 (US)
(72) Inventor: Hstch, Charles Terrance, Minden, NV Nevada 89423 (US); Campbell, Lam Arthur, Minden, NV Nevada 89423 (US); Sheikmann, Boris Leonid, Minden, NV Nevada 89423 (US); Whitefield II, Charles David, Minden, NV Nevada (US)
(74) Representative: Picker, Madeline Margaret

(57) **Abstract**

A display assembly (118) for use with a monitoring system (100) is provided. The display assembly includes a communication interface (202) that is configured to receive hazardous gas (102) data indicative of a concentration level for at least one gaseous component. Moreover, the display assembly also includes a processor (210) that is coupled to the communication interface, wherein the processor is configured to generate at least one image based on the hazardous gas data. The display assembly also includes a display media (218) coupled to the processor, wherein the display media is configured to present the image to a user in real-time. The display assembly is positioned against the user such that the display assembly is movable with the user and the user is enabled to monitor hazardous gases within a location (108) while the user moves about the location.

## Description

### BACKGROUND OF THE INVENTION

The field of the invention relates generally to monitoring systems and, more particularly, to monitoring systems for use in monitoring hazardous gases.

In many industrial facilities, such as cogeneration facilities and power plants, the potential for hazardous gases to be emitted into the environment and surrounding areas exists. For example, at least some known coal plants may generate and emit various levels of carbon monoxide (CO). Moreover, at least some known cogeneration facilities and power plants may include one or more engines that emit hazardous gases. Moreover, processing plants, such as chemical processing plants, can produce flammable and/or explosive gases, such as aromatic hydrocarbons, and toxic gases, such as hydrogen sulfide (H₂S). Accordingly, monitoring hazardous gases within such systems is essential.

To detect the presence of hazardous gases within such industrial facilities, at least some known sensor or monitoring systems may be used. At least some known monitoring systems use at least one sensor to detect the presence of at least one gaseous component. The sensor then transmits data received to a display device that enable a user to monitor the gaseous component within the facility. However, generally known systems may not provide real-time data, as the user may be required to go to a different location to view the display device. Moreover, while at least some known monitoring systems can be worn on the body to enable a user to have direct knowledge of the presence of hazardous gases, such monitoring systems are limited to only providing an audio signal when a predefined threshold is reached. More specifically, such monitoring systems do not provide a display that enables a user to monitor other conditions of hazardous gases, such as concentration levels and/or the different types of hazardous gases present within the facility. Accordingly, such monitoring systems do not enable a user to react more quickly to a developing danger relating to hazardous gases.

### BRIEF DESCRIPTION OF THE INVENTION

In one embodiment, a display assembly for use in a monitoring system is provided. The display assembly includes a communication interface that is configured to receive hazardous gas data indicative of a concentration level for at least one gaseous component. Moreover, the display assembly also includes a processor that is coupled to the communication interface, wherein the processor is configured to generate at least one image based on the hazardous gas data. The display assembly also includes a display media coupled to the processor, wherein the display media is configured to present the image to a user in real-time. The display assembly is positioned against the user such that the display assembly is movable with the user and the user is enabled to monitor hazardous gases within a location while the user moves about the location.

In another embodiment, a monitoring system is provided. The monitoring system includes a sensor assembly including at least one sensor that is configured to detect at least one gaseous component and to generate at least one signal representative of hazardous gas data based on the detection of the gaseous component. The hazardous gas data is indicative of a concentration level for the gaseous component. Moreover, the monitoring system includes a display assembly that is communicatively coupled to the sensor assembly. The display assembly includes a communication interface that is configured to receive the hazardous gas data. Moreover, the display assembly also includes a processor that is coupled to the communication interface, wherein the processor is configured to generate at least one image based on the hazardous gas data. The display assembly also includes a display media coupled to the processor, wherein the display media is configured to present the image to a user in real-time. Moreover, the display assembly is positioned against the user such that the display assembly is movable with the user and the user is enabled to monitor hazardous gases within a location while the user moves about the location.

In yet another embodiment, a method of monitoring hazardous gases is provided. The method includes positioning a display assembly against a user such that the display assembly is movable with the user and the user is enabled to continuously monitor hazardous gases within a location while the user moves about the location. Moreover, hazardous gas data is received, wherein the hazardous gas data is indicative of a concentration level for at least one gaseous component. Further, at least one image is generated based on the hazardous gas data. The image is presented to the user in real-time via a display media.

### BRIEF DESCRIPTION OF THE DRAWINGS

Fig. 1 is a block diagram of an example monitoring system;
Fig. 2 is a schematic perspective view of an example display assembly that may be used with the monitoring system shown in Fig. 1;
Fig. 3 is a block diagram of an alternative display assembly that may be used with the monitoring system shown in Fig. 1; and
Fig. 4 is a flow diagram of an example method for use in monitoring hazardous gases using the display assembly shown in Fig. 2.

### DETAILED DESCRIPTION OF EMBODIMENTS OF THE INVENTION

The example methods and systems described herein overcome at least some disadvantages associated with known systems for use in monitoring hazardous gases within an industrial facility. In particular, the embodiments described herein provide a monitoring system that includes a display assembly that receives hazardous gas data indicative of a concentration level of at least one gaseous component, and that generates at least one image based on the hazardous gas data. The display assembly also includes a display media that presents the image to a user. Moreover, the display assembly may be positioned against the user such that the display assembly is movable with the user and the user may be able to continuously monitor hazardous gases within their current location even while the user moves about the location. As such, the monitoring system disclosed herein is not limited to only providing an audio signal when a predefined threshold is reached, but rather also provides additional information, such as concentration levels and/or can identify different types of hazardous gases within the facility. Accordingly, such monitoring systems enable a user to react more quickly to a developing danger relating to hazardous gases.

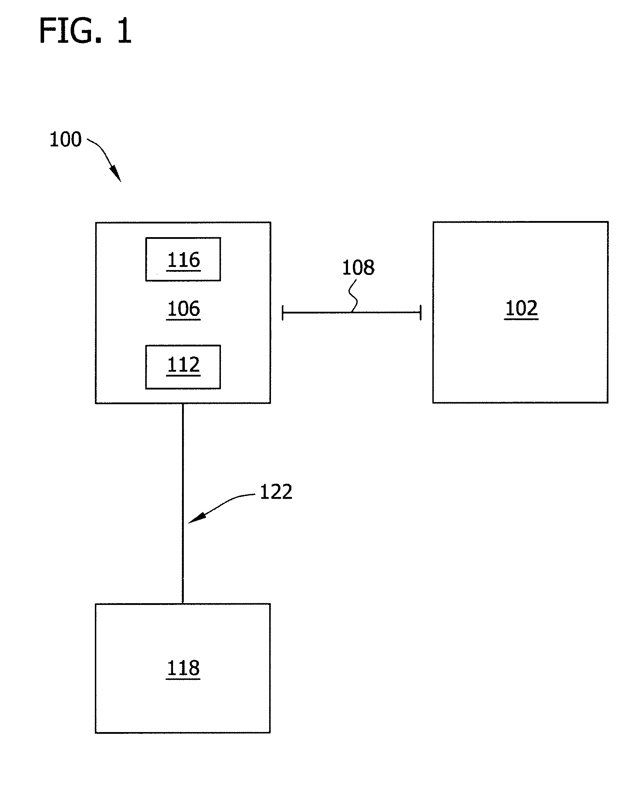

Fig. 1 illustrates an example monitoring system 100 that may be used to enable a user (not shown) to monitor hazardous gases within a location (not shown) in an industrial facility (not shown), such as a cogeneration facility and/or power plant. More specifically, in the example embodiment, monitoring system 100 enables a user to monitor various types of hazardous gases being emitted from a hazardous gas source 102, such as, but not limited to, a steam turbine engine and/or a gas turbine engine. While the example embodiment describes a monitoring system being used in an industrial facility, the present invention is not limited to an industrial facility, and one of ordinary skill in the art will appreciate that the current invention may be used in connection with any facility that may contain hazardous gases.

In the example embodiment, monitoring system 100 includes a sensor assembly 106 that is spaced a distance 108 from hazardous gas source 102. Sensor assembly 106 includes at least one transducer or sensor 112. More specifically, in the example embodiment, sensor assembly 106 includes a plurality of sensors 112 that each detect the presence of at least one gaseous component (not shown) within distance 108 from source 102. More specifically, in the example embodiment, each sensor 112 detects a particular type of gaseous component and detects a concentration level for each gaseous component. Alternatively, sensors 112 may be configured to detect various other parameters of hazardous gases that enable sensor assembly 106 and/or monitoring system 100 to function as described herein.

In the example embodiment, sensor assembly 106 also includes a sensor communication interface 116 that enables sensor assembly 106 to communicate with at least one other component of monitoring system 100. More specifically, monitoring system 100 includes a display assembly 118, and communication interface 116 is coupled to display assembly 118 via network 122. It should be noted that, as used herein, the term "couple" is not limited to a direct mechanical, communication, and/or an electrical connection between components, but may also include an indirect mechanical, communication and/or electrical connection between multiple components.

In the example embodiment, sensor assembly 106 communicates with display assembly 118 using a wireless communication means, such as radio frequency (RF), e.g., FM radio and/or digital audio broadcasting, an Institute of Electrical and Electronics Engineers (IEEE®) 802.11 standard (e.g., 802.11(g) or 802.11(n)), the Worldwide Interoperability for Microwave Access (WIMAX®) standard, a cellular phone technology (e.g., the Global Standard for Mobile communication (GSM)), a satellite communication link, and/or any other suitable communication means. WIMAX is a registered trademark of WiMax Forum, of Beaverton, Oregon. IEEE is a registered trademark of Institute of Electrical and Electronics Engineers, Inc., of New York, New York. Alternatively, sensor assembly 106 may communicate with display assembly 118 using a wired network connection (e.g., Ethernet or an optical fiber).

In the example embodiment, communication interface 116 enables sensor assembly 106 to communicate with display assembly 118. More specifically, in the example embodiment, communication interface 116 receives information from each sensor 112, such as hazardous gas data associated with the particular type of gaseous component detected and a concentration level for each gaseous component detected. Moreover, communication interface 116 transmits a signal representative of the hazardous gas data to display assembly 118 based on information received from each sensor 112.

In the example embodiment, display assembly 118 receives the hazardous gas data and presents the hazardous gas data to the user in the form of at least one image. In the example embodiment, display assembly 118 may be positioned against the user, such as against the body (not shown) of the user, such that the display assembly 118 is movable with the user and the user is enabled to continuously monitor hazardous gases within their current location, such as distance 108, even while the user moves about the location. For example, display assembly 118 may be worn or held by the user.

In the example embodiment, sensor assembly 106 may also be positioned against the user, such as against the body of the user. For example, sensor assembly 106 may be worn or held by the user. Alternatively, sensor assembly 106 may not be positioned against the user and may be located anywhere within the industrial facility.

During operation, in the example embodiment, as the user approaches hazardous gas source 102, if any hazardous gases are emitted from hazardous gas source 102, such gases will be detected when the user is within distance 108. Each sensor 112 detects the presence of at least one gaseous component by detecting a particular type of gaseous component and detecting a concentration level for each gaseous component. The hazardous gas data is transmitted to communication interface 116 such that the hazardous gas data can be received by display assembly 118, wherein the data is presented to the user in real-time. In the example embodiment, because display assembly 118 and sensor assembly 106 are positioned against the user, hazardous gas data received by display assembly 118 may change based on the location of the user.

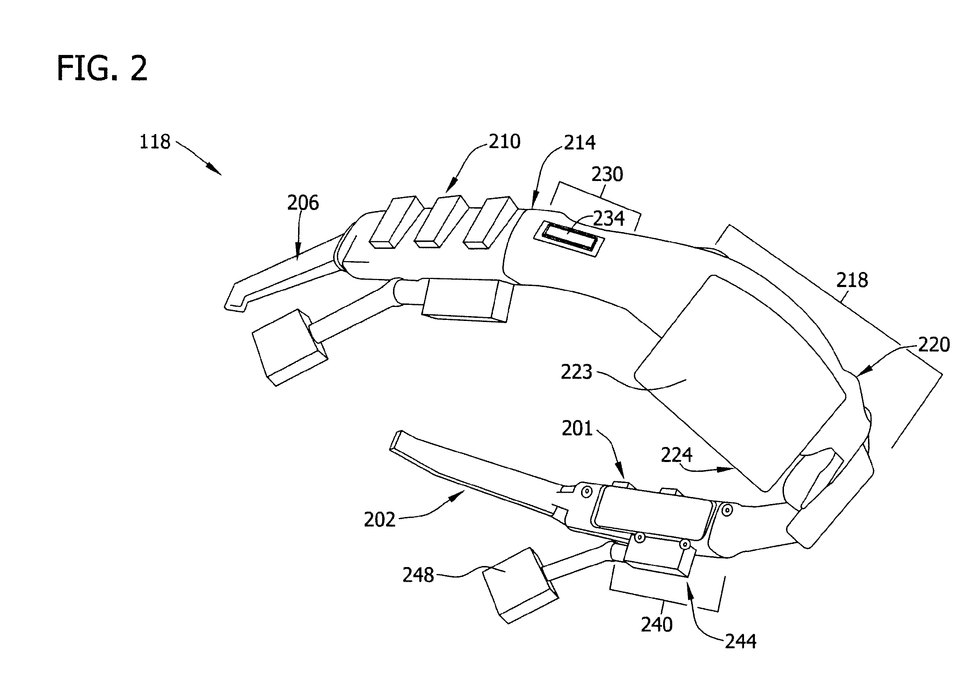

Fig. 2 is a schematic diagram of an example display assembly 118 that may be used with monitoring system 100 (shown in Fig. 1). In the example embodiment, display assembly 118 is worn by a user (not shown) and may be positioned against the user, such as against the body of the user. More specifically, in the example embodiment, display assembly 118 is a pair of eyeglasses worn by the user. Alternatively, display assembly 118 may be any form such that display assembly may be against the user and that enables display assembly 118 and/or monitoring system 100 to function as described herein.

In the example embodiment, display assembly 118 includes a battery 201 that provides power to display assembly 118. In the example embodiment, battery 201 is a rechargeable lithium-ion battery 201. Alternatively, battery 201 may be any other lithium-based battery or any other type of battery that enables display assembly 118 to function as described herein.

Display assembly 118 includes a communication interface 202 that receives hazardous gas data from sensor assembly 106 (shown in Fig. 1). More specifically, in the example embodiment, sensor communication interface 116 (shown in Fig. 1) is coupled to communication interface 202 via network 122 (shown in Fig. 1 ). Communication interface 116 transmits a signal representative of the hazardous gas data received from each sensor 112 (shown in Fig. 1) to communication interface 202. In the example embodiment, communication interface 202 is an antenna, such as, for example, an antenna that may be used for wireless radio communication. Alternatively, communication interface 202 may be any other type of communication module that enables display assembly 118 and/or monitoring system 100 to function as described herein.

In the example embodiment, display assembly 118 also includes a receiver 206 that is communicatively coupled to communication interface 202. In the example embodiment, receiver 206 is a wireless receiver that receives hazardous gas data from communication interface 202 via a wireless data connection. Receiver 206 transmits the hazardous gas data to a processor 210 coupled to communication interface 202 and to receiver 206 via a system bus (not shown). Processor 210 is also coupled to a memory device 214 via the system bus.

In some embodiments, executable instructions are stored in memory device 214. Display assembly 118 is programmable to perform one or more operations described herein by programming processor 210. For example, processor 210 may be programmed by encoding an operation as one or more executable instructions and providing the executable instructions in memory device 214. Processor 210 may include one or more processing units (e.g., in a multi-core configuration). In the example embodiment, processor 210 is programmed to continuously generate at least one image based on the hazardous gas data processor continues to receive from sensor assembly 106. More specifically, in the example embodiment, processor 210 is programmed to generate an image that includes the name of at least one gaseous component detected.

Processor 210 is programmed to generate an image that includes a graphical representation of a concentration level for at least one gaseous component detected. Processor 210 is also programmed to generate a textual warning if the concentration level exceeds a predefined threshold level. For example, the textual warning may flash according to a sequence of time intervals when presented to a user. In the example embodiment, the textual warning is provided prior to the concentration level of the gaseous component exceeding the predefined threshold level. Alternatively, processor 210 may be programmed to generate any other image(s) that enable display assembly 118 and/or monitoring system 100 to function as described herein. Moreover, in the example embodiment, processor 210 is programmed to generate an audio output based on the concentration level for the gaseous component exceeding the predefined threshold value.

As used herein, the term "processor" refers generally to any programmable system including systems and microcontrollers, reduced instruction set circuits (RISC), application specific integrated circuits (ASIC), programmable logic circuits (PLC), and any other circuit or processor capable of executing the functions described herein. The above represent examples, and thus are not intended to limit in any way the definition and/or meaning of the term "processor."

Processor 210 may include, but is not limited to, a general purpose central processing unit (CPU), a graphics processing unit (GPU), a microcontroller, a reduced instruction set computer (RISC) processor, an application specific integrated circuit (ASIC), a programmable logic circuit (PLC), and/or any other circuit or processor capable of executing the functions described herein. The methods described herein may be encoded as executable instructions embodied in a computer readable medium, including, without limitation, a storage device and/or a memory device. Such instructions, when executed by processor 210, cause processor 210 to perform at least a portion of the methods described herein. The above examples are example only, and thus are not intended to limit in any way the definition and/or meaning of the term processor.

Memory device 214 enables information such as executable instructions and/or other data to be stored and retrieved. Memory device 214 may include one or more computer readable media, such as, without limitation, dynamic random access memory (DRAM), static random access memory (SRAM), a solid state disk, and/or a hard disk. Memory device 214 may be configured to store, without limitation, executable instructions, configuration data, geographic data (e.g., topography data and/or obstructions), utility network equipment data, and/or any other type of data.

In the example embodiment, memory device 214 stores the hazardous gas data received from sensor assembly 106 and stores the images that are generated by processor 210. Moreover, in the example embodiment, memory device 214 may include random access memory (RAM), which can include non-volatile RAM (NVRAM), magnetic RAM (MRAM), ferroelectric RAM (FeRAM) and other forms of memory. Memory device 214 may also include read only memory (ROM), flash memory and/or Electrically Erasable Programmable Read Only Memory (EEPROM). Any other suitable magnetic, optical and/or semiconductor memory, by itself or in combination with other forms of memory, may be included in memory device 214. Memory device 214 may also be, or include, a detachable or removable memory, including, but not limited to, a suitable cartridge, disk, CD ROM, DVD or USB memory. Alternatively, memory device 214 may be a database. The term "database" refers generally to any collection of data including hierarchical databases, relational databases, flat file databases, object-relational databases, object oriented databases, and any other structured collection of records or data that is stored in a computer system. The above represent examples only, and thus are not intended to limit in any way the definition and/or meaning of the term database. Examples of databases include, but are not limited to only including, Oracle® Database, MySQL, IBM® DB2, Microsoft® SQL Server, Sybase®, and PostgreSQL. However, any database may be used that enables the systems and methods described herein. (Oracle is a registered trademark of Oracle Corporation, Redwood Shores, California; IBM is a registered trademark of International Business Machines Corporation, Armonk, New York; Microsoft is a registered trademark of Microsoft Corporation, Redmond, Washington; and Sybase is a registered trademark of Sybase, Dublin, California.)

A display media 218 and a display adaptor 220 are also coupled to processor 210 via the system bus. In the example embodiment, display media 218 includes least one screen 223 within at least one lens 224. More specifically, in the example embodiment, display media 218 includes two lenses 224, wherein each lens 224 has one screen 223 within. In the example embodiment, lenses 224 are polarizing lenses. Alternatively, lenses 224 may be any type of lens that enables display media 218 to function as described herein. Moreover, in the example embodiment, display media 218 presents the images generated by processor 210 to the user. More specifically, in the example embodiment, display media 218 includes a visual display, such as a cathode ray tube (CRT), a liquid crystal display (LCD), an organic LED (OLED) display, and/or an "electronic ink" display. As such, in the example embodiment, the user is enabled to see the images on at least one of the lenses 224.

In the example embodiment, display assembly 118 also includes a user interface 230 that is coupled to processor 210 via the system bus. User interface 230 receives any information suitable for use with the methods described herein. More specifically, in the example embodiment, the user can input the various images the user would like displayed on display media 218 and the user can input whether the user would like to receive information via an audio signal. Moreover, in the example embodiment, user interface 230 includes a touch sensitive panel 234. Alternatively, user may include, for example, a keyboard, a pointing device, a mouse, a stylus, a touch pad, a touch screen, a gyroscope, an accelerometer, a position detector. Display assembly 118 also includes an audio input interface 240 coupled to processor via the system bus. In the example embodiment, audio input interface 240 includes a microphone 244 to enable the user to communicate with a third party.

Moreover, in the example embodiment, display assembly 118 includes an audio output device 248 that is coupled to processor 210 via the system bus. In the example embodiment, audio output device 248 is an audio adapter and/or a speaker. Alternatively, audio output device 248 may be any type of device that enables display assembly 118 and/or monitoring system 100 to function as described herein. In the example embodiment, audio output device 248 is configured to receive an output from processor 210 when the gaseous component exceeds the predefined threshold level and to generate an audio signal based on the output received. More specifically, audio output device 248 is configured to generate an audio signal based on the concentration level of at least one gaseous component exceeding a predefined threshold level. Audio output device 248 is configured to transmit the audio signal to the user. In the example embodiment, the audio signal is an audio alarm that may annunciate an actual concentration level and/or warning. Alternatively, audio signal may be any type of audio signal that enables display assembly 118 and/or monitoring system 100 to function as described herein.

During operation, in the example embodiment, as the user wearing display assembly 118 approaches hazardous gas source 102 (shown in Fig. 1), if any hazardous gases are emitted from hazardous gas source 102, such gases will be detected when the user is within distance 108 (shown in Fig. 1). Each sensor 112 (shown in Fig. 1) detects the presence of at least one gaseous component by detecting the particular type of gaseous component and by detecting a concentration level for each gaseous component. The hazardous gas data is transmitted to communication interface 116.

The hazardous gas data is transmitted to display assembly 118 and is received by communication interface 202. Communication interface 202 transmits the hazardous gas data to receiver 206, which transmits the data to processor 210 and to memory device 214 to enable the hazardous gas data to be stored. Processor 210 continuously generates a plurality of images based on the hazardous gas data that communication interface 202 continues to receive. More specifically, processor 210 generates an image that includes the name of each gaseous component detected and a graphical representation of a concentration level of each gaseous component detected. If the concentration level exceeds a predefined threshold level that is programmed in processor 210, then processor 210 generates an image that includes a textual warning.

Display media 218 continuously presents information to the user based on the input the user provides to user interface 230. More specifically, the user can input whether the information is presented via a visual output and/or audio output. If the user chooses to receive the information via a visual output, processor 210 continuously transmits the plurality of images to display media 218. The user can then visually identify various parameters of the hazardous gases within the industrial facility. More specifically, the user will be presented with an image that includes the name of each gaseous component detected and a graphical representation of a concentration level for each gaseous component detected. Moreover, if the concentration level exceeds a predefined threshold level, then the user will see an image that includes a textual warning. These images will continue to change as each of the parameters change while the user moves about a location within the industrial facility.

If the user chooses to receive additional information via an audio output, processor 210 transmits an audio output to audio output device 248 when the gaseous component exceeds a predefined threshold level. Audio output device 248 generates an audio signal based on the output received. More specifically, audio output device 248 generates an audio signal based on a concentration level for a gaseous component exceeding a predefined threshold level. Audio output device 248 then transmits the audio signal to the user.

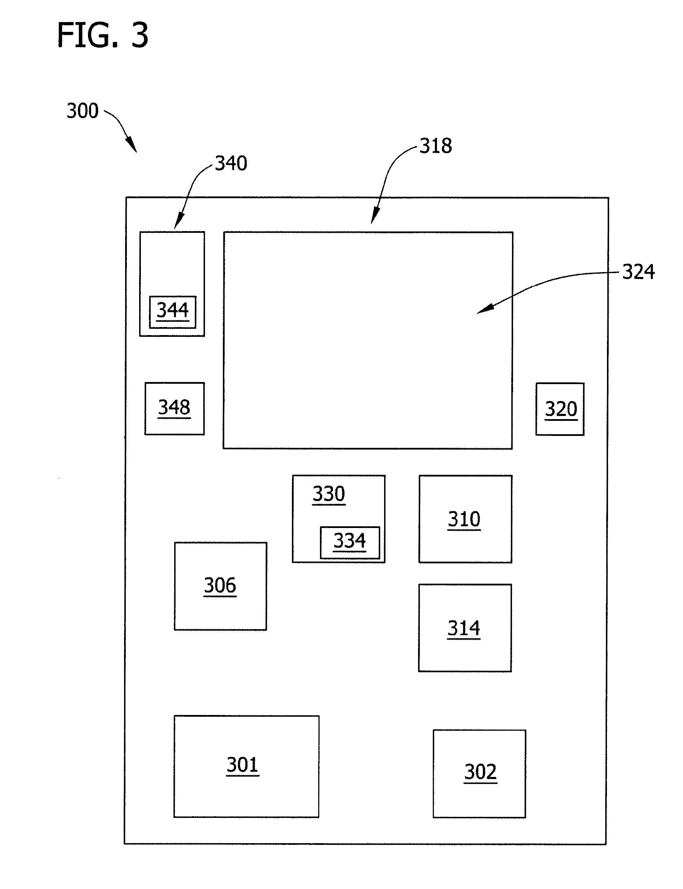

Fig. 3 illustrates an alternative embodiment of a display assembly 300 that may be used with monitoring system 100 (shown in Fig. 1) rather than display assembly 118 (shown in Figs. 1 and 2). In the example embodiment, display assembly 300 is held or retained by a user (not shown) such that display assembly 300 is positioned against the user, such as against the body (not shown) of the user. More specifically, display assembly 300 is a handheld computing device, such as a smart phone. Alternatively, display assembly 300 may be any form such that display assembly 300 may be positioned against the user and that enables display assembly 300 and/or monitoring system 100 to function as described herein.

Moreover, in the example embodiment, display assembly 300 includes a battery 301 to provide power to display assembly 300. In the example embodiment, battery 301 is a rechargeable lithium-ion battery 301. Alternatively, battery 301 may be any other lithium-based battery or any other type of battery that enables display assembly 300 and/or monitoring system to function as described herein.

Display assembly 300 includes a communication interface 302 that receives hazardous gas data from sensor assembly 106 (shown in Fig. 1). More specifically, in the example embodiment, sensor communication interface 116 (shown in Fig. 1) is coupled to communication interface 302 via network 122 (shown in Fig. 1 ). Communication interface 116 transmits a signal representative of the hazardous gas data received from each sensor 112 (shown in Fig. 1) to communication interface 302. Moreover, in the example embodiment, communication interface 302 is a short-range wireless communication channel such as BLUETOOTH®. BLUETOOTH is a registered trademark of Bluetooth SIG, Inc. of Kirkland, Washington. Alternatively, communication interface 302 may be any other type of communication module that enables display assembly 300 and/or monitoring system 100 to function as described herein.

In the example embodiment, display assembly 300 also includes a receiver 306 that is communicatively coupled to communication interface 302. More specifically, in the example embodiment, receiver 306 is a wireless receiver that receives the hazardous gas data from communication interface 302 via a wireless data connection (not shown). Receiver 306 transmits the hazardous gas data to a processor 310 coupled to communication interface 302 and receiver 306 via a system bus (not shown). Processor 310 is also coupled to a memory device 314 via the system bus.

In some embodiments, executable instructions are stored in memory device 314. Display assembly 300 is programmable to perform one or more operations described herein by programming processor 310. For example, processor 310 may be programmed by encoding an operation as one or more executable instructions and providing the executable instructions in memory device 314. Processor 310 may include one or more processing units (e.g., in a multi-core configuration). In the example embodiment, processor 310 is programmed to continuously generate at least one image based on the hazardous gas data processor continues to receive from sensor assembly 106.

More specifically, in the example embodiment, processor 310 is programmed to generate an image that includes the name of at least one gaseous component detected. Processor 310 is programmed to generate an image that includes a graphical representation of a concentration level for at least one gaseous component detected. Processor 310 is also programmed to generate a textual warning if the concentration level exceeds a predefined threshold level. More specifically, in the example embodiment, the textual warning is provided prior to the concentration level of the gaseous component exceeding the predefined threshold level. Alternatively, processor 310 may be programmed to generate any other images that enable display assembly 300 and/or monitoring system 100 to function as described herein. In the example embodiment, processor 310 is programmed to generate an audio output based on the concentration level for the gaseous component exceeding the predefined threshold value.

Moreover, processor 310 may include, but is not limited to, a general purpose central processing unit (CPU), a graphics processing unit (GPU), a microcontroller, a reduced instruction set computer (RISC) processor, an application specific integrated circuit (ASIC), a programmable logic circuit (PLC), and/or any other circuit or processor capable of executing the functions described herein. The methods described herein may be encoded as executable instructions embodied in a computer readable medium, including, without limitation, a storage device and/or a memory device. Such instructions, when executed by processor 310, cause processor 310 to perform at least a portion of the methods described herein. The above represent examples only, and thus are not intended to limit in any way the definition and/or meaning of the term processor.

Memory device 314 enables information such as executable instructions and/or other data to be stored and retrieved. Memory device 314 may include one or more computer readable media, such as, without limitation, dynamic random access memory (DRAM), static random access memory (SRAM), a solid state disk, and/or a hard disk. Memory device 314 may be configured to store, without limitation, executable instructions, configuration data, geographic data (e.g., topography data and/or obstructions), utility network equipment data, and/or any other type of data.

In the example embodiment, memory device 314 stores the hazardous gas data received from sensor assembly 106 and stores the images that are generated by processor 310. Moreover, in the example embodiment, memory device 314 may include random access memory (RAM), which can include non-volatile RAM (NVRAM), magnetic RAM (MRAM), ferroelectric RAM (FeRAM) and other forms of memory. Memory device 314 may also include read only memory (ROM), flash memory and/or Electrically Erasable Programmable Read Only Memory (EEPROM). Any other suitable magnetic, optical and/or semiconductor memory, by itself or in combination with other forms of memory, may be included in memory device 314. Memory device 314 may also be, or include, a detachable or removable memory, including, but not limited to, a suitable cartridge, disk, CD ROM, DVD or USB memory. Alternatively, memory device 314 may be a database. The term "database" refers generally to any collection of data including hierarchical databases, relational databases, flat file databases, object-relational databases, object oriented databases, and any other structured collection of records or data that is stored in a computer system. The above represent examples only, and thus are not intended to limit in any way the definition and/or meaning of the term database. Examples of databases include, but are not limited to only including, Oracle® Database, MySQL, IBM® DB2, Microsoft® SQL Server, Sybase®, and PostgreSQL. However, any database may be used that enables the systems and methods described herein. (Oracle is a registered trademark of Oracle Corporation, Redwood Shores, California; IBM is a registered trademark of International Business Machines Corporation, Armonk, New York; Microsoft is a registered trademark of Microsoft Corporation, Redmond, Washington; and Sybase is a registered trademark of Sybase, Dublin, California.)

A display media 318 and a display adaptor 320 are also coupled to processor 310 via the system bus. In the example embodiment, display media 318 includes a display screen 324. Moreover, in the example embodiment, display media 318 presents the images generated by processor 310 to the user. More specifically, in the example embodiment, display media 318 includes a visual display, such as a cathode ray tube (CRT), a liquid crystal display (LCD), an organic LED (OLED) display, and/or an "electronic ink" display. As such, in the example embodiment, the user is enabled to see the images on screen 324.

In the example embodiment, display assembly 300 also includes a user interface 330 that is coupled to processor 310 via the system bus. User interface 330 receives any information suitable for use with the methods described herein. More specifically, in the example embodiment, the user can input the various images the user would like displayed on display media 318 and the user can input whether the user would like an audio signal as well. Moreover, in the example embodiment, user interface 330 includes a keyboard 334. Alternatively, user may include, for example, a pointing device, a mouse, a stylus, a touch pad, a touch screen, a gyroscope, an accelerometer, a position detector. Display assembly 300 also includes an audio input interface 340 coupled to processor via the system bus. In the example embodiment, audio input interface 340 includes a microphone 344 to enable the user to communicate with a third party.

In the example embodiment, display assembly 300 includes an audio output device 348 that is coupled to processor 310 via the system bus. In the example embodiment, audio output device 348 is an audio adapter and/or a speaker. Alternatively, audio output device 348 may be any type of device that enables display assembly 300 and/or monitoring system 100 to function as described herein. In the example embodiment, audio output device 348 is configured to receive an output from processor 310 when the gaseous component exceeds the predefined threshold level. Audio output device 348 is configured to generate an audio signal based on the output received. More specifically, audio output device 348 is configured to generate an audio signal based on the concentration level for at least one gaseous component exceeding a predefined threshold level. Audio output device 348 is configured to transmit the audio signal to the user that may annunciate an actual concentration level and/or warning. In the example embodiment, the audio signal is an audio alarm. Alternatively, audio signal may be any type of audio signal that enables display assembly 300 and/or monitoring system 100 to function as described herein.

During operation, in the example embodiment, as the user holding display assembly 300 approaches hazardous gas source 102 (shown in Fig. 1), if any hazardous gases are emitted from hazardous gas source 102, such gases will be detected when the user is within distance 108 (shown in Fig. 1). Each sensor 112 (shown in Fig. 1) detects the presence of at least one gaseous component by detecting the type of gaseous component and by detecting a concentration level for each gaseous component. This hazardous gas data is transmitted to communication interface 116 (shown in Fig. 1) and then transmitted to display assembly 300. More specifically, the hazardous gas data is received by communication interface 302. Communication interface 302 transmits the hazardous gas data to receiver 306, which transmits the data to processor 310 and to memory device 314 to enable the hazardous gas data to be stored. Processor 310 continuously generates a plurality of images based on the hazardous gas data communication interface 302 continues to receive. More specifically, processor 310 generates an image that includes the name of each gaseous component detected and a graphical representation of a concentration level of each gaseous component detected. If the concentration level exceeds a predefined threshold level that is programmed in processor 310, then processor 310 generates an image that includes a textual warning.

Display media 318 continuously presents information to the user based on the input the user provides to user interface 330. More specifically, the user can input whether the information is presented via a visual output and/or audio output. If the user chooses to receive the information via a visual output, processor 310 continuously transmits the plurality of images to display media 318. The user can then visually identify various parameters of the hazardous gases within the industrial facility. More specifically, the user will be presented with an image that includes the name of each gaseous component detected and a graphical representation of a concentration level of each gaseous component detected. Moreover, if the concentration level exceeds a predefined threshold level, then the user will see an image that includes a textual warning. These images will continue to change as each of the each parameters change while the user moves about a location within the industrial facility.

If the user chooses to receive additional information via an audio output, processor 310 transmits an audio output to audio output device 348 when the gaseous component exceeds a predefined threshold level. Audio output device 348 generates an audio signal based on the output received. More specifically, audio output device 348 generates an audio signal based on a concentration level for a gaseous component exceeding a predefined threshold level. Audio output device 348 then transmits the audio signal to the user.

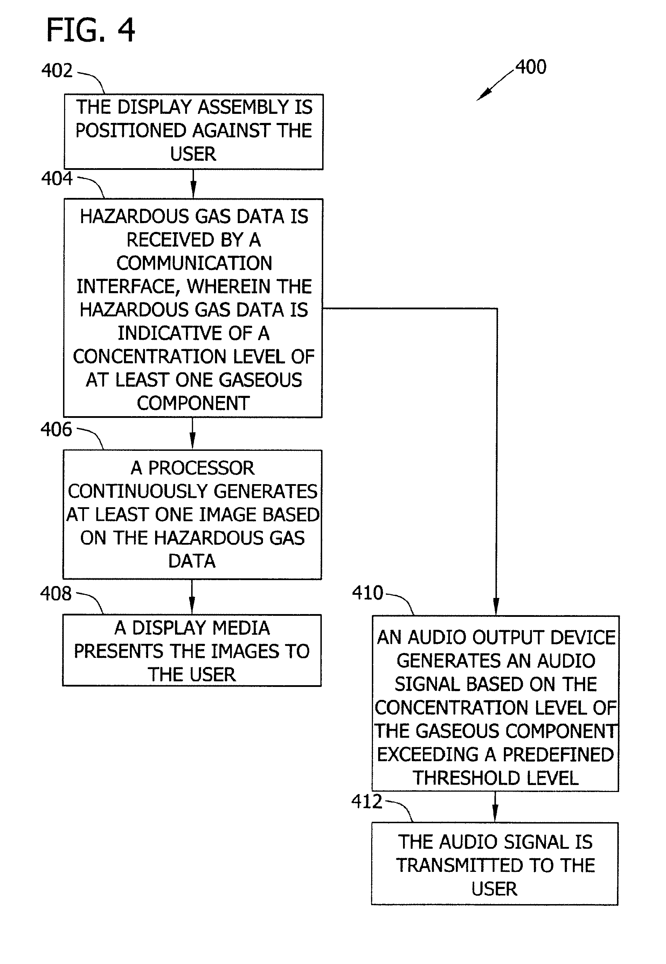

Fig. 4 is a flow chart that illustrates an example method 400 for use in monitoring hazardous gases using a display assembly, such as display assembly 118 (shown in Figs. 1 and 2). Alternatively, method 400 may use display assembly 300 (shown in Fig 3). In the example embodiment, display assembly 118 is positioned 402 against the user. More specifically, display assembly 118 is positioned against the body of the user such that display assembly 118 is movable with the user and the user is enabled to continuously monitor hazardous gases within a location, even while the user moves about the location. Hazardous gas data is received 404 by a communication interface 202 (shown in Fig. 2). Such hazardous gas data is indicative of at least a concentration level of at least one gaseous component. A processor 210 (shown in Fig. 2) continuously generates 406 at least one image based on the hazardous gas data. More specifically, in the example embodiment, the images generated include the name of each gaseous component detected and a graphical representation of a concentration level for each gaseous component detected. Moreover, if the concentration level exceeds a predefined threshold level that is programmed in processor 210, then an image that includes a textual warning is generated. These images will continue to change based on the each of the parameters changing while the user moves about the location.

A display media 218 (shown in Fig. 2) presents 408 the images to the user. Moreover, in the example embodiment, an audio output device 248 (shown in Fig. 2) generates 410 an audio signal based on a concentration level of a gaseous component exceeding a predefined threshold level. The audio signal is then transmitted 412 to the user.

As compared to known systems and methods that are used to monitor hazardous gases, the above-described embodiments of methods and apparatus enable a user to react more quickly to a developing danger relating to hazardous gases. In particular, the embodiments described herein provide a monitoring system that includes a display assembly that receives hazardous gas data indicative of a concentration level of at least one gaseous component, and that continuously generates at least one image based on the hazardous gas data. The display assembly also includes a display media that presents the image to a user. Moreover, the display assembly may be positioned against the user such that the display assembly is movable with the user and the user may be able to continuously monitor hazardous gases within their current location even while the user moves about the location. As such, the monitoring system disclosed herein is not limited to only providing an audio signal when a predefined threshold is reached, but rather also provides additional information, such as concentration levels and/or can identify different types of hazardous gases within the facility.

Technical features of the systems and methods described herein includes at least one of: (a) positioning a display assembly against a user such that the display assembly is movable with the user and the user is enabled to continuously monitor hazardous gases within a location while the user moves about the location; (b) receiving hazardous gas data indicative of a concentration level for at least one gaseous component; (c) generating at least one image based on hazardous gas data; and (d) presenting in real-time, via a display media, at least one image to the user.

Example embodiments of systems and methods for use in monitoring hazardous gases are described above in detail. The systems and methods are not limited to the specific embodiments described herein, but rather, components of the systems and/or steps of the methods may be utilized independently and separately from other components and/or steps described herein. For example, the systems may also be used in combination with other systems and methods, and are not limited to practice with only the systems as described herein. Rather, the example embodiment can be implemented and utilized in connection with many other applications.

Although specific features of various embodiments of the invention may be shown in some drawings and not in others, this is for convenience only. In accordance with the principles of the invention, any feature of a drawing may be referenced and/or claimed in combination with any feature of any other drawing.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A display assembly (118) for use with a monitoring system (100), said display assembly comprising:
a communication interface (202) configured to receive hazardous gas data indicative of a concentration level for at least one gaseous component;
a processor (210) coupled to said communication interface, wherein said processor is configured to generate at least one image based on the hazardous gas data; and
a display media (218) coupled to said processor, wherein said display media is configured to present the at least one image to a user in real-time, said display assembly is positioned against the user such that the display assembly is movable with the user and the user is enabled to monitor hazardous gases within a location (108) while the user moves about the location.

2. A display assembly (118) in accordance with Claim 1, wherein said display media (218) comprises at least one lens (224), and/or said display media (218) comprises at least one display screen (324).

3. A display assembly (118) in accordance with Claim 1 or 2, wherein the at least one image includes at least one of a graphical representation of the concentration level for the at least one gaseous component and a name of the at least one gaseous component.

4. A display assembly (118) in accordance with Claim 1, 2 or 3, wherein the at least one image provides a textual warning if the concentration level for the at least one gaseous component exceeds a predefined threshold level.

5. A display assembly (118) in accordance with Claim 4, wherein the textual warning is provided prior to the concentration level for the at least one gaseous component exceeding the predefined threshold level.

6. A display assembly (118) in accordance with any one of Claims 1 to 5, further comprising an audio output device (248) coupled to said processor (210), wherein said audio output device is configured to generate an audio signal based on the concentration level for the at least one gaseous component exceeding a predefined threshold level, said audio output device is configured to transmit the audio signal to the user.

7. A monitoring system (100) comprising:
a sensor assembly (106) comprising at least one sensor (112) configured to detect at least one gaseous component and to generate at least one signal representative of hazardous gas data based on the detection of at least one gaseous component, wherein the hazardous gas data is indicative of a concentration level for the at least on gaseous component;
a display assembly (118) communicatively coupled to said sensor assembly, said display assembly comprising:
a communication interface (202) configured to receive the hazardous gas data;
a processor (210) coupled to said communication interface, wherein said processor is configured to generate at least one image based on the hazardous gas data; and
a display media (218) coupled to said processor, wherein said display media is configured to present the at least one image to a user in real-time, said display assembly is positioned against a user such that the display assembly is movable with the user and the user is enabled to monitor hazardous gases within a location (108) while the user moves about the location.

8. A monitoring system (100) in accordance with Claim 7, wherein said sensor assembly (106) is positioned against the user such that the sensor assembly is movable with the user.

9. A monitoring system (100) in accordance with Claim 7 or Claim 8, wherein said display media (218) comprises at least one of a lens (224) and a display screen (223).

10. A monitoring system (100) in accordance with Claim 7, 8 or 9, wherein the at least one image includes at least one of a graphical representation of the concentration level for the at least one gaseous component and a name of the at least one gaseous component.

11. A monitoring system (100) in accordance with any one of Claims 7 to 10, wherein the at least one image provides a textual warning if the concentration level for the at least one gaseous component exceeds a predefined threshold level, wherein the textual warning is provided prior to the concentration level for the at least one gaseous component exceeding the predefined threshold level.

12. A monitoring system in accordance with any one of Claims 7 to 11, wherein said display assembly (118) further comprises an audio output device coupled to said processor, said audio output device (248) is configured to generate an audio signal based on the concentration level for the at least one gaseous component exceeding a predefined threshold level, wherein said audio output device is configured to transmit the audio signal to the user.

13. A method of monitoring hazardous gases, said method comprising:
positioning a display assembly (118) against a user such that the display assembly is movable with the user and the user is enabled to monitor hazardous gases within a location while the user moves about the location;
receiving hazardous gas data indicative of a concentration level for at least one gaseous component;
generating at least one image based on the hazardous gas data; and
presenting in real-time, via a display media, the at least one image to the user.

14. A method in accordance with Claim 13, wherein presenting in real-time further comprises one of:
presenting in real-time, via at least one screen within at least one lens, the at least one image to the user;
presenting in real-time, via at least one display screen, the at least one image to the user;
presenting in real-time, via a display media, at least one of a graphical representation of the concentration level for the at least one gaseous component and a name of the at least one gaseous component; and
presenting in real-time, via a display media, a textual warning if the concentration level for the at least one gaseous component exceeds a predefined threshold level, preferably comprising presenting the textual warning prior to the concentration level for the at least one gaseous component exceeding the predefined threshold level.

15. A method in accordance with Claim 13 or Claim 14, further comprising:
generating via an audio output device (248) an audio signal based on the concentration level for the at least one gaseous component exceeding a predefined threshold level; and
transmitting the audio signal to the user.
